(19) **Europäisches Patentamt / European Patent Office / Office européen des brevets**

(11) **EP 2 053 862 B1**

(12) **EUROPEAN PATENT SPECIFICATION**

(45) Date of publication and mention
of the grant of the patent:
**29.01.2014 Bulletin 2014/05**

(51) Int Cl.:
*H04N 7/18* (2006.01)   *G02B 23/24* (2006.01)
*G02B 7/08* (2006.01)   *A61B 1/00* (2006.01)

(21) Application number: **08017029.3**

(22) Date of filing: **26.09.2008**

(54) **Imaging apparatus and endoscope system**

Bildgebungsvorrichtung und Endoskopsystem

Appareil d'imagerie et système endoscopique

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.10.2007 JP 2007275582**

(43) Date of publication of application:
**29.04.2009 Bulletin 2009/18**

(73) Proprietor: **FUJIFILM Corporation
Tokyo (JP)**

(72) Inventor: **Takahira, Masayuki
Tokyo (JP)**

(74) Representative: **Klunker . Schmitt-Nilson . Hirsch
Patentanwälte
Destouchesstrasse 68
80796 München (DE)**

(56) References cited:
JP-A- 2003 140 030   US-A1- 2007 063 048
US-A1- 2007 156 021   US-A1- 2007 223 898
US-B1- 6 236 881

Printed by Jouve, 75001 PARIS (FR)

**Description**

This application is based on and claims priority under 35 U.S.C §119 from Japanese Patent Application No. 2007-275582, filed on October 23, 2007, the entire disclosure of which is herein incorporated by reference.

BACKGROUND OF THE INVENTION

1. Field of the Invention

The present invention relates to an imaging apparatus and endoscope system that takes a moving image and obtains a still image in accordance with its operation.

2. Description of Related Art

In the field of medical treatment, endoscope systems are broadly used to insert an elongate tube (optical probe) having a mirror, an imaging device, etc. at the tip to a body interior of a subject and observe tumors or blood clots by taking an image of the body interior of the subject. By directly taking an image of the body interior of the subject, it is possible to grasp a color, shape, etc. of a seat of a disease difficult to see by a radiographic image without inflicting external damages to the subject, which enables to easily obtain information required for deciding a treatment policy or so.

The endoscope system has a freeze function to extract a frame image and produce a still image in timing with the freeze operation from the user, in addition to the ordinary imaging function to take a frame image repeatedly at a time interval and display on the monitor a moving image the frame images continue successively. The physician usually moves the optical probe while looking the moving image displayed on the monitor, presses the operation button and makes a freeze operation when the optical probe is moved to a desired observation point, and records a generated still image in a recording medium so as to be utilized in later diagnosis. However, even if freeze operation is performed in a state the subject is placed stationary, the observation point delicately moves due to the movement of organs, blood, etc. as long as taking an image of an interior of a living body. For this reason, image blur is possibly caused in the still image taken, which requires the repeated freeze operations many times in order to obtain a still image useful for diagnosis with a result of inflicting burden on the subject and the user.

In this connection, a technique is devised that, when receiving a freeze instruction during taking a moving image, a subject movement is detected by comparing a plurality of frame images taken within a time with reference to the time the freeze instruction has been received so that a frame image least in subject movement can be determined as the optimal still image (see Japanese Patent No. 2902662 and JP-B-8-34577).

However, the techniques described in Japanese Patent No. 2902662 and JP-B-8-34577 can relieve the blur of the still image due to beat but cannot relieve the image blur resulting from the high-frequency vibrations in the movement shorter in time than the frame rate or the image obscurity resulting from out of focus. The endoscope apparatus is not easy to focus because its focal length is as short as several millimeters and the depth of field is shallow in enlarging observation. Moreover, high-frequency vibrations possibly arise in the optical probe due to resonance with the motor, etc. In the technique described in Japanese Patent No. 2902662 and JP-B-8-34577, it is impossible to detect a deterioration of image quality responsible for such a cause. Thus, still images are eventually required to be taken many times.

From US 2007/0156021 A1 an AF process for endoscopes comprising a fluid lens is known.

Meanwhile, that is not limited only to the endoscope but to arise generally in the field of imaging apparatuses where still images are extracted in accordance with freeze operation.

SUMMARY OF THE INVENTION

An object of the invention is to provide an imaging apparatus and endoscope system capable of easily obtaining a quality still image free from the occurrence of out-of-focus.

According to an aspect of the invention, there is provided an imaging apparatus including:

an imaging section that repeatedly takes an image of a subject to obtain a plurality of subject images;
a contrast calculating section that calculates a contrast of the image with respect to  each of the plurality of subject images;
a time trigger generating section that receives an operation during repeatedly taking the image at the imaging section and issues a time trigger representing a time at which the operation is received; and
a display section that displays a subject image when the time trigger is issued from the time trigger generating section, in which the contrast of the subject image calculated by the contrast calculating section is the highest among a part of the subject images having image-taking times in a time region including the time represented by the time trigger.

According to this imaging apparatus, a subject image is to be displayed that is highest in contrast out of part of the subject images having image-taking times in a time region including a time represented by the time trigger. The use of image contrast makes it possible to properly determine an image blur or out-of-focus due to high-frequency vibrations that could not have been determined in the related-art method for detecting a subject movement in an image, and to easily obtain a quality subject image.

In the imaging apparatus, the contrast calculating section calculates a contrast of a subject image each time the subject image is obtained at the imaging section, the image apparatus may further includes:

a storage section that stores a certain number of subject images in a newer order among the subject images obtained at the imaging section; and

a subject image selecting section that selects, each time the contrast is calculated by the contrast calculating section, a subject image highest in the contrast among the subject images stored in the storage section as a candidate for a subject image to be displayed on the display section, and that determines, when the time trigger is issued from the time trigger section, the subject image selected as the candidate for a subject image to be displayed on the display section, and

the display section may display the subject image determined by the subject image selecting section.

The process time from issuing a time trigger to displaying a subject image can be shortened by calculating a contrast each time a subject image is obtained and selecting a subject image highest in contrast out of the subject images stored in the storage section.

In the imaging apparatus, each time a subject image is obtained by the imaging section, the display section may display the obtained subject image, and when the time trigger is issued from the time trigger generating section, the display section may display the subject image highest in the contrast among the part of the subject images.

According to this imaging apparatus, the user is allowed to easily obtain a quality subject image in a desired observation point or observation state by issuing a time trigger in desired timing while confirming, on a screen, a plurality of subject images obtained through repeatedly taking an image of the subject.

In the imaging apparatus, the contrast calculating section may obtain contrasts at a plurality of points in a subject image and calculate a contrast of the subject image based on the obtained contrasts.

According to this imaging apparatus, the contrast of the subject image entirety can be calculated easily.

In the imaging apparatus, the contrast calculating section may obtain contrasts at a plurality of points in a subject image and calculate a contrast of the subject image based on contrasts equal to or greater than a lower limit out of the obtained contrasts.

The disadvantage that the noises, etc. occurring in the subject image have effects upon calculating a contrast of the subject image entirety can be relieved by calculating a subject image contrast through utilizing only the contrasts equal to or greater than the lower limit.

In the imaging apparatus, the contrast calculating section may obtain contrasts at a plurality of points in a subject image and calculate a contrast of the subject image based on the obtained contrasts after correcting those exceeding an upper limit to a value within the upper limit.

The disadvantage that the excessively high contrast at the boundary, etc. between a point where light is being illuminated and a point where light is not being illuminated has effects upon calculating a contrast of the subject image entirety can be relieved by correcting the contrast exceeding the predetermined upper limit to a value within the upper limit.

In the imaging apparatus, the imaging section may obtain an image of a subject having a light arrival area where subject light arrives from the subject and a light non-arrival area where the subject light does not arrive surrounding the light arrival area, and the contrast calculating section may calculate a contrast within the light arrival area as a contrast of the subject image.

For example, in an endoscope or the like for taking an image of a body interior of a subject, there is a case that subject light arrives only at a partial area of an image obtained as a subject image while it is pitch-dark in the outer side of that area, due to the structure of the imaging section. In such a case, the difference in lightness is great between the area where light is arriving and the area it is pitch-dark, wherein contrast is excessively high at and around the boundary thereof. According to this imaging apparatus, because a contrast within the light illuminated area is calculated, contrasts at desired observation points themselves are calculated to select a quality subject image.

Meanwhile, according to an aspect of the invention, there is provided an endoscope system including:

a light source that emits light;
a light conducting path that guides the light emitted from the light source and illuminates light to a subject;

an imaging section that repeatedly takes an image of a subject to obtain a plurality of subject images;

a contrast calculating section that calculates a contrast of the image with respect to each of the plurality of subject images;

a time trigger generating section that receives an operation during repeatedly taking the image at the imaging section and issues a time trigger representing a time at which the operation is received; and

a display section that displays a subject image when the time trigger is issued from the time trigger generating section, in which the contrast of the subject image calculated by the contrast calculating section is the highest among a part of the subject images having image-taking times in a time region including the time represented by the time trigger.

According to this endoscope system, a quality subject image can be obtained that is free from the occurrence of image blur or out-of-focus due to high-frequency vibrations.

BRIEF DESCRIPTION OF THE DRAWINGS

The features of the invention will appear more fully upon consideration of the exemplary example of the invention, which are schematically set forth in the drawings, in which:

Fig. 1 is a schematic arrangement view of an endoscope system applied to an exemplary embodiment of the present invention;

Fig. 2 is a schematic functional block diagram of the endoscope system;

Fig. 3 is a functional configuration diagram of a freeze processing section shown in Fig. 2;

Fig. 4 is a flowchart showing a series of process flow from pressing the freeze button to displaying a still image on the monitor;

Fig. 5 is a figure showing a relationship between the imaging area of the optical probe and the arrival area of light;

Figs. 6A and 6B are figures for explaining a way of calculating a contrast at a subject-of-calculation pixel;

Fig. 7 is a concept figure of an evaluation memory; and

Figs. 8A-8C are figures for explaining the image quality of a still image to be frozen by the endoscope system in the embodiment.

DETAILED DESCRIPTION OF THE EXEMPLARY EMBODIMENTS

According to an exemplary embodiment of the invention, a quality still image can be easily obtained that is free from the occurrence of out-of-focus, etc.

An exemplary embodiment in the present invention is explained in the following with reference to the drawings.

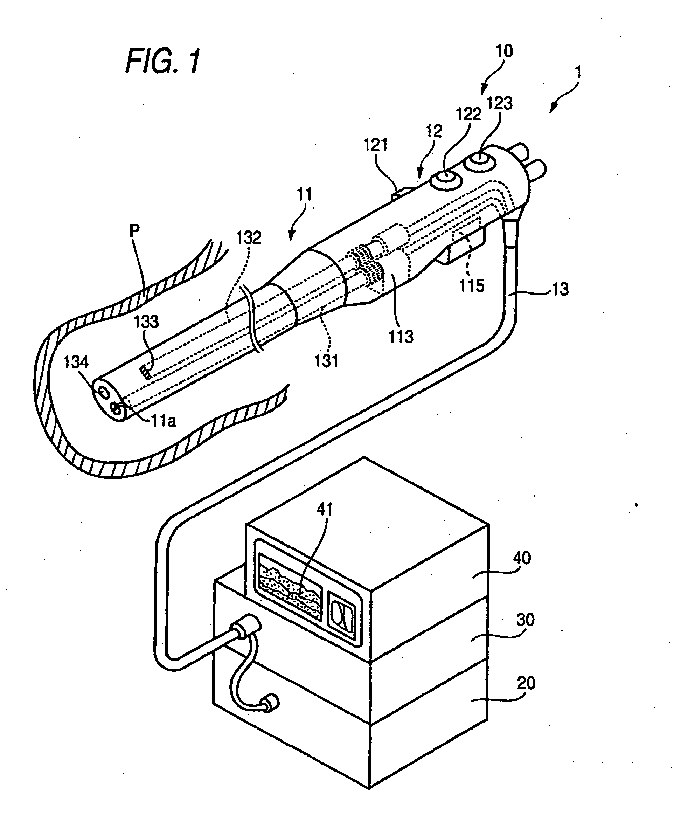

Fig. 1 is a schematic arrangement view of an endoscope system to which an exemplary embodiment of the invention is applied.

An endoscope system 1 shown in Fig. 1 includes an optical probe 10 that introduces and illuminates light to a body interior of a subject P and generates an image signal on the basis of the reflection light thereof, a light source device 20 that emits light, an image processing device 30 that performs image processing on the image obtained at the optical probe 10 and produces a medical image which a body interior of the subject P is taken, and a display device 40 that displays on a monitor 41 the medical image produced by the image processing device 30. The endoscope system 1 is mounted with a usual imaging function that takes a frame image repeatedly at a time interval and displays a moving image the frame images continue successively on the monitor 41, and a freeze function that extracts a frame image in timing with operation and generates a still image. The display device 40 corresponds to an example of a display section and the light source device 20 to an example of a light source.

The optical probe 10 includes an elongate probe body 11 having flexibility, a controller 12 for operating the probe body 11, and a light/signal guide 13 connecting among the light source device 20, the image processing device 30 and the optical probe 10. In the following, the optical probe 10 is explained with the end to be inserted in a body interior of the subject P taken as a front end and the end opposite to the front end as a rear end.

The controller 12 is provided with a curvature operating lever 121 for causing curvature in the probe body 11, a freeze button 122 for obtaining a still image by freeze processing, and a color adjusting button 123 for adjusting the color of an image being displayed. The freeze button 122 corresponds to an example of a time trigger generating section.

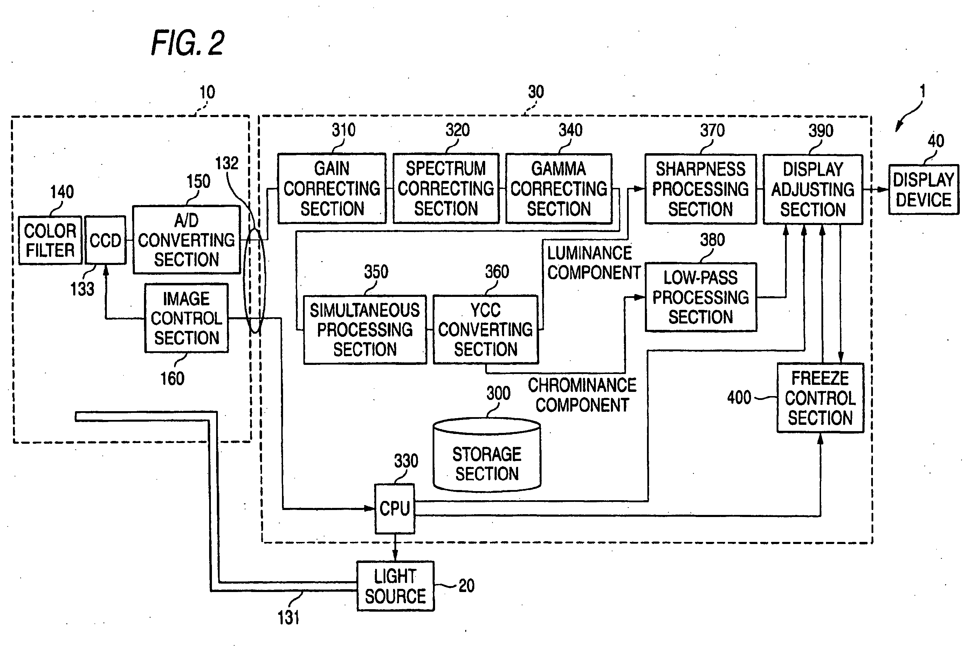

The light/signal guide 13 includes a light guide 131 that conducts light and a signal line 132 that transmits a signal. The light guide 131 is connected at its rear end to the light source device 20 so that it guides the light emitted from the light source device 20 to an interior of the probe body 11 and illuminates the light toward the subject P through an illumination window 11a provided at the front end of the probe body 11. The light guide 131 corresponds to an example of a light conducting path. The signal line 132 has a front end attached with a CCD 133 and a rear end connected to the image processing device 30. The reflection light, which the light illuminated through the illumination window 11a of the light guide 131 is reflected in the body interior of the subject P, is collected by an optical member 134 provided at the front end of the probe body 11 and received by the CCD 133 to generate a taken image by the reflection light. The CCD 133 is arranged with a plurality of light-receiving elements so that image data represented with a plurality of pixels can be generated by receiving light at the plurality of light-receiving elements. In the present embodiment, the CCD 133 is fixed with a color filter (see Fig. 2) that R, G and B colors are arranged in a regular color pattern in positions corresponding, respectively, to the plurality of light-receiving elements. By receiving the light passed through the color filter at the CCD 133, a color mosaic image is produced that R, G and B colored pixels are arranged in the same pattern as the color pattern of the color filter.

The generated color mosaic image is conveyed to the image processing device 30 through the signal line 132 and subjected to image processing at the image processing device 30.

Fig. 2 is a schematic functional block diagram of the endoscope system 1.

Note that, in Fig. 2, the main elements related to image signal generation only are shown by omitting the monitor 41, the controller 12 of the optical probe 10 and so on.

The light source device 20 shown also in Fig. 1 is for issuing white light and is to be controlled by an overall control section (CPU) 330 of the image processing device 30.

The optical probe 10 is provided with a color filter 140 that arranges R, G and B colors in a mosaic form with a regular color pattern, an A/D converting section 150 that converts the analog image signal generated by the CCD 133 into a digital image signal, an image control section 160 that controls the processing of various elements of the optical probe 10 and so on, in addition to the CCD 133 shown also in Fig. 1. The combination of the CCD 133 and the A/D converting section 150 corresponds to an example of an imaging section.

The image processing device 30 is provided with a storage section 300 that stores a still image, etc. obtained by pressing the freeze button 122, a gain correcting section 310 that corrects the gain of an image sent from the optical probe 10, a spectrum correcting section 320 that corrects the spectral characteristic of the optical probe 10 including the CCD 133, a gamma correcting section 340 that performs gray-level correction on the image, a simultaneous processing section 350 that generates a color image represented with a color mixture of R, G and B, three colors at pixels by interpolating, with use of surrounding pixels, the other color components (e.g. B and G colors) than the color component (e.g. R color) possessed by the pixels of the color mosaic image generated at the optical probe 10, a YCC converting section 360 that resolves the image with a luminance component Y and a chrominance component Cr, Cb, a sharpness processing section 370 that performs sharpness processing on the luminance component, a low-pass processing section 380 that removes a high-frequency component from the chrominance component Cr, Cb and reduces false colors, a display adjusting section 390 that converts the YCC image formed by a luminance component Y and a chrominance component Cr, Cb into an image displayable on the monitor 41 of the display device 40, a freeze processing section 400 that selects a frame image highest in contrast out of the frame images taken within a time from the time the freeze button 122 shown in Fig. 1 is pressed, a CPU 330 that controls the overall processing of the optical probe 10 and image processing device 30, and so on. The storage section 300 corresponds to a storage section.

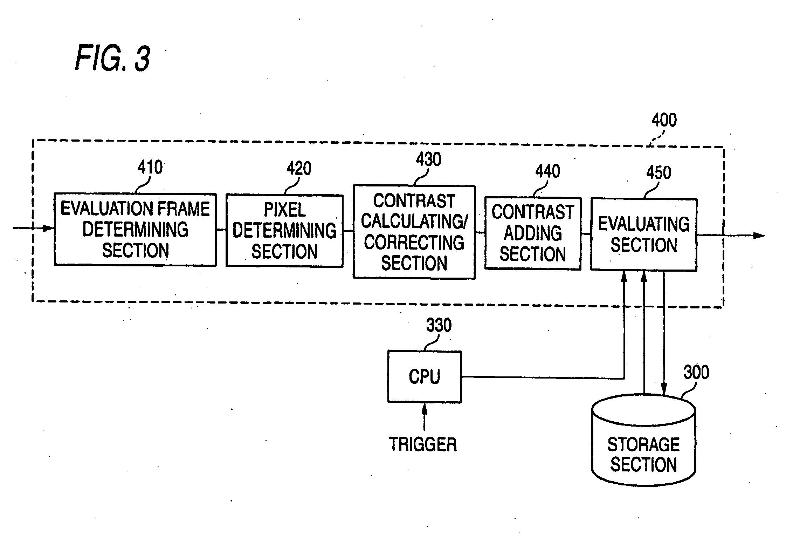

Fig. 3 is a functional configuration diagram of the freeze processing section 400 shown in Fig. 2.

The freeze processing section 400 is provided with an evaluation-frame determining section 410 that determines whether or not each frame image is a subject of evaluation as to contrast for a plurality of frame images conveyed repeatedly, a pixel determining section 420 that determines whether or not each pixel is a subject of calculation as to contrast for a plurality of pixels in a frame image determined as a subject of evaluation, a contrast calculating/correcting section 430 that calculates a contrast at a pixel that is a subject of calculation and corrects the contrast higher than an upper limit value into the upper limit value, a contrast adding section 440 that calculates the total sum of contrast over subject-of-calculation pixels in an amount of one frame image, and a evaluating section 450 that estimates the frame image greatest in the total sum of contrast out of the subject-of-evaluation frame images taken within a predetermined time. The contrast calculating/correcting section 430 corresponds to a contrast calculating section and the evaluating section 450 corresponds to an example of a subject-image selecting section.

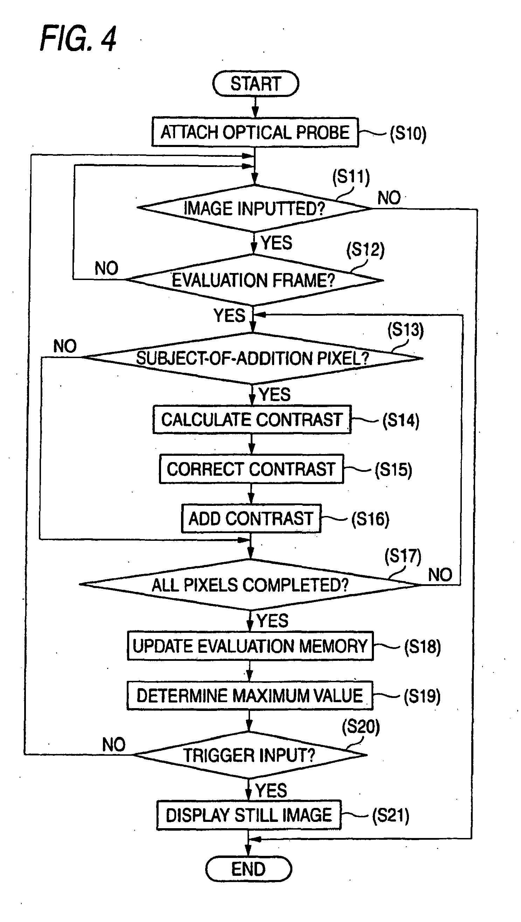

Fig. 4 is a flowchart showing a series of process flow from pressing the freeze button 122 up to displaying a still image on the monitor 41.

From now on, a series of process flow up to generating a still image is explained according to the flowchart.

At first, an optical probe 10 in a size suited for a subject observation point is selected, and the selected optical probe 10 is attached to the light source device 20 and image processing device 30 (step S10 in Fig. 4).

When the optical probe 10 is attached, identifying information for identifying the optical probe 10 is conveyed from the image control section 160 of the optical probe 10 shown in Fig. 2 to the CPU 330 of the image processing device 30.

The storage section 300 previously stores the identifying information for an optical probe 10, various parameter values for executing the image processing suited for the optical probe 10, and a scope diameter of the optical scope 10 shown in Fig. 1, with association one with another. The CPU 330 sets the various parameters associated with the identification information conveyed from the optical probe 10 to the gain correcting section 310, the spectrum correcting section 320, the gamma correcting section 340, the simultaneous processing section 350, the YCC converting section 360, the sharpness processing section 370, the low pass processing section 380 and the display adjusting section 390, and notifies a scope diameter to the freeze processing section 400.

The image processing device 30 is previously prepared with a setting screen on which setting is to be made for a thin-out interval of a subject-of-evaluation frame image which the total sum of contrast is to be calculated at the freeze processing section 400 and for a thin-out interval of subject-of-calculation pixels on which contrast is to be calculated. When the user sets up a subject-of-evaluation frame image and a thin-out interval of the subject-of-calculation pixels according to the setting screen displayed on the monitor 41, setting is notified from the CPU 330 to the freeze processing section 400. In this example, explanation is made on the assumption that the thin-out intervals of subject-of-evaluation frame images and subject-of calculation pixels are both set at "1 (every other)".

After completing the various settings, actual imaging of the subject is started. When the optical probe 10 is inserted in the body interior of the subject P, the light emitted from the light source device 20 is introduced to the front end of the optical probe 10 by means of the light guide 131 and illuminated to the body interior of the subject P through the illumination window 11a. The reflection light, which the light emitted from the light source device 20 is reflected in the body interior of the subject P, travels through the color filter 140 and is received by the CCD 133 where a imaging image is generated (step S11 in Fig. 4: Yes). The generated imaging image is digitized at the A/D converting section 150 and then conveyed into the image processing device 30 through the signal line 132. As mentioned above, the optical probe 10 repeatedly takes a frame image at a time interval (frame rate), to generate a moving image that the frame images continued successively. Namely, a plurality of frame images are successively inputted to the image processing device 30.

The frame images, inputted into the image processing device 30, are corrected for gain at the gain correcting section 310, subjected to spectrum correcting process at the spectrum correcting section 320 and subjected to gray-level correcting process at the gamma correcting section 340, followed by being conveyed to the simultaneous processing section 350.

The simultaneous processing section 350 performs simultaneous process on the frame image, a mosaic-colored image, and converts it into a color image that pixels are represented with color mixtures of R, G and B, three, colors. In the YCC converting section 360, the converted frame image is color-separated with a chrominance component Cr, Cb and a luminance component Y. The chrominance component Cr, Cb, due to color separation, is conveyed to the low-pass processing section 380 and the luminance component Y is conveyed to the sharpness processing section 370.

In the sharpness processing section 370, image visibility is adjusted by performing sharpness process on the luminance component Y. The luminance component Y, sharpness-processed, is conveyed to the display adjusting section 390. Meanwhile, in the low-pass processing section 380, the chrominance component Cr, Cb is removed of its high-frequency component and subjected to false-color reduction process. The chrominance component Cr, Cb whose false colors were reduced is conveyed to the display adjusting section 390 where it is combined with the luminance component Y conveyed from the sharpness processing section 370.

The combined frame image is conveyed to the freeze processing section 400 and subjected to color adjustment process for the monitor 41 at the display adjusting section 390. By performing image process, in order, on the frame images successively generated at the optical probe 10 and conveying those to the display device 40, a moving image is displayed in real time on the monitor 41.

Meanwhile, the frame image conveyed to the freeze processing section 400 is determined whether or not it is a subject-of-evaluation frame on which the total sum of contrast is evaluated, in the evaluation-frame determining section 410 shown in Fig. 3 (step S12 in Fig. 4). In this example, because the subject-of-evaluation frame has a thin-out interval set at "1", the evaluation-frame determining section 410 determines the successively conveyed frame images, thinned-out every other, as a subject-of-evaluation frame image (step S12 in Fig. 4: Yes). The subject-of-evaluation frame image is conveyed to the pixel determining section 420.

In the pixel determining section 420, a plurality of pixels constituting the subject-of-evaluation frame image conveyed from the evaluation-frame determining section 410 are each determined whether or not a subject-of-calculation pixel on which contrast is to be calculated (step S13 in Fig. 4). In the present embodiment, determination is made based on the scope diameter of the optical probe 10 and the thin-out interval of subject-of-calculation pixels established by the user.

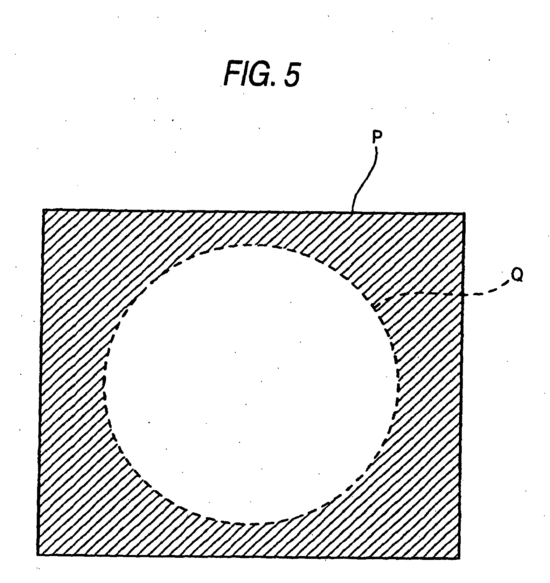

Fig. 5 is a figure showing a relationship between an imaging area of the optical probe 10 and a light arrival area.

The CCD 133 is to obtain a subject image within an imaging area P surrounded by the outer solid lines in Fig. 5 whereas the light emitted from the light source device 20 and introduced to the illumination window 11a of the optical probe 10 is to reach only within a light arrival area Q surrounded by the broken line in Fig. 5, wherein it is pitch-dark in the area excepting the light arrival area. For this reason, contrast increases on pixels at and around the boundary between the light arrival area Q where light arrives and the area where light does not arrive, which results in a possibility not to accurately determine whether or not out-of-focus is occurring in the imaging image. In the present embodiment, when the optical probe 10 is attached, the scope diameter of the optical probe 10 has been notified to the freeze processing section 400. The pixel determining section 420 determines the pixels, included in an area inner by a range (four pixels in the present embodiment) than the light arrival area Q where light arrives, as subject-of-calculation pixels by thinning-out at a thin-out interval (every other, in this embodiment) as set up by the user. The light arrival area Q corresponds to an example of a light arrival area and the area (hatched area) excluding the light arrival area Q from the imaging area P corresponds to an example of a light non-arrival area.

The determination result is conveyed to the contrast calculating/correcting section 430.

In the contrast calculating/correcting section 430, contrast at the subject-of-calculation pixel is calculated (step S 14 in Fig. 4).

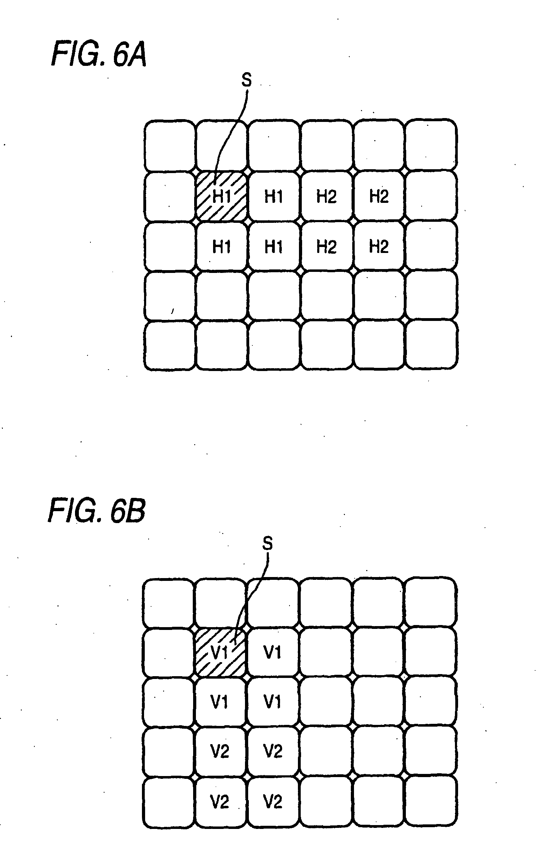

Figs. 6A and 6B are figures for explaining a way to calculate contrast at the subject-of-calculation pixel.

Fig. 6A is a figure showing a concept of horizontal contrast at a subject-of-calculation pixel S and Fig. 6B is a figure showing a concept of vertical contrast at the subject-of-calculation pixel S.

In calculating a contrast at the subject-of-calculation pixel S, a group H1 of four peripheral pixels including the subject-of-calculation pixel S and a group H2 of four peripheral pixels arranged horizontally to the peripheral pixel group H1 are detected as shown in Fig. 6A. Furthermore, a group V1 of four peripheral pixels including the subject-of-calculation pixel S and a group V2 of four peripheral pixels arranged vertically to the peripheral pixel group V 1 are detected as shown in Fig. 6 partB. Next, if taking an X axis horizontally and a Y axis vertically in Fig. 6 with reference to the subject-of-calculation pixel S as an origin, the contrast I_s at the subject-of-calculation S is calculated by the following equation, with using pixel values I(x, y) of the pixels.

$$I\_s = Abs\left(\sum_{H2} I(x, y) - \sum_{H1} I(x, y)\right) + Abs\left(\sum_{V2} I(x, y) - \sum_{V1} I(x, y)\right) \qquad (1)$$

When the contrast I_s at the subject-of-calculation pixel S is calculated, the contrast I_s is corrected to a value equal to or smaller than a threshold T (step S15 in Fig. 4). Where taking an image with light illumination to a diseased part in a dark body interior, false color possibly occurs during taking an image because color is spatially changed by high-frequency waves. This results in a possibility of increasing contrast in a false-colored image region. For this reason, in the case that the calculated contrast I_s is in excess of a threshold T, the contrast I_s at the subject-of-calculation pixel S is reduced to the threshold T.

The calculated contrast I_s is conveyed to the contrast adding section 440. The contrast adding section 440 is prepared with a contrast summation variable previously set at "0". The contrast adding section 440 adds the contrast summation variable with the contrast I_s at the subject-of-calculation pixel S (step S16 in Fig. 4).

Determining a subject-of-calculation pixel S (step S13 in Fig. 4), calculating a contrast at the subject-of-calculation pixel S (step S14 in Fig. 4), correcting for contrast (step S 15 in Fig. 4) and addition of contrast (step S16 in Fig. 4) are performed on all the pixels constituting the frame image (step S 17 in Fig. 4).

After completing the contrast calculation/addition process in an amount of one frame (step S 17 in Fig. 4: Yes), the contrast adding section 440 notifies the value of contrast summation variable to the evaluating section 450 and initializes the value of contrast summation variable to "0". The value of contrast summation variable conveyed to the evaluating section 450 is representative of the total sum of contrast over one frame image, which is a contrast evaluation value for evaluating the contrast over the frame image entirety. The evaluating section 450 stores a frame image associatively with the value of contrast summation variable (contrast evaluation value) conveyed from the contrast adding section 440, in the evaluation memory prepared in the storage section 300, and updates the evaluation memory (step S 18 in Fig. 4).

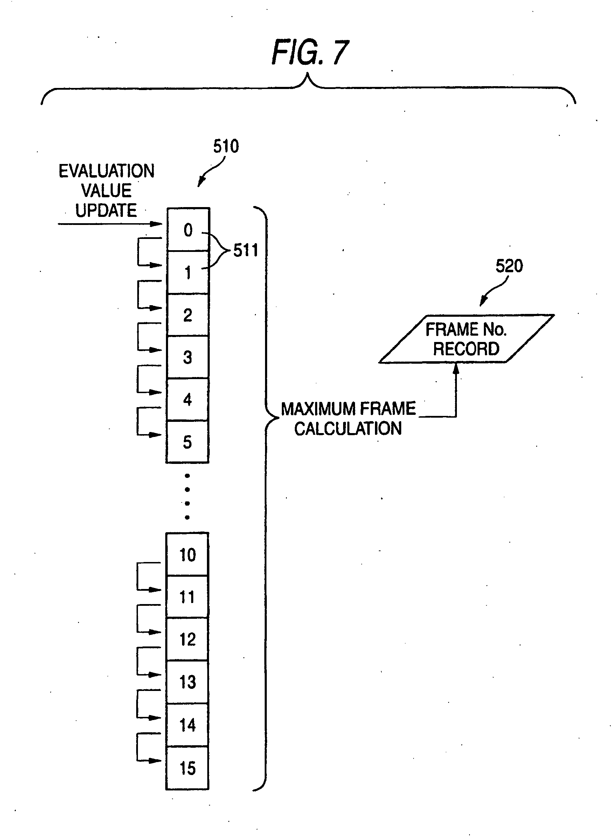

Fig. 7 is a concept figure of the evaluation memory.

In the present embodiment, the storage section 300 is prepared with an evaluation memory 510 that stores a frame image and a contrast evaluation value associatively and a maximum memory 520 that stores an identification number (frame number) of a frame image maximum in contrast evaluation value out of the frame images being stored in the evaluation memory 510. The evaluation memory 510 is provided with a plurality of storage areas 511 to which a series of number, i.e. 0 - N (N = 15 in the example of Fig. 7), are given wherein each of the storage areas 511 is stored with each one set of a frame image and a contrast evaluation value.

In the evaluating section 450, when a new set of a frame image and a contrast evaluation value is conveyed, the sets being stored in a plurality of storage areas 511 are stored onto the one-succeeding storage areas 511. In this case, the set being stored in the N-th (fifteenth in the example in Fig. 7) storage area 511 greatest in number is overwritten and deleted by the set having been stored in the one-preceding, (N-1)-th (fourteenth in the example in Fig. 7) storage area 511. When completing the movement of the sets already stored, the new set conveyed from the contrast calculating section 440 is stored in the 0-th storage area 511 smallest in number (step S1 in Fig. 4).

Furthermore, in the evaluating section 450, the greatest set in contrast evaluation value is searched out of the sets being stored in the plurality of storage areas 511 (step S 19 in Fig. 4). The frame number of the frame image associated with the maximum contrast evaluation value is stored in the maximum memory 520.

Each time a frame image is conveyed to the freeze processing section 400, it is determined whether or not it is a subject-of-evaluation frame. In the case it is a subject-of-evaluation frame, a contrast evaluation value is calculated and a maximum value is determined as to contrast evaluation value whereby the maximum memory 520 is always allowed to store the frame number of a frame image maximum in contrast evaluation value out of the frame images having been taken in the past within a time with reference to the present time.

Here, when the freeze button 122 shown in Fig. 1 is pressed by the user, a trigger is inputted to the CPU 390 so that a still-image output instruction is notified from the CPU 390 to the evaluating section 450 (step S20 in Fig. 4: Yes). The evaluating section 450, when the still-image output instruction is notified, acquires the frame image attached with the frame number being stored in the maximum memory 520 out of the frame images being stored in the plurality of storage areas 510. The acquired frame image is conveyed as a still image to the display device 40 through the display adjusting section 390.

In the display device 40, the still image conveyed from the display adjusting section 390 is displayed on the monitor 41 (step S21 in Fig. 4). When the user confirms the still image displayed on the monitor 41 and operates the save switch (not shown), the still image is recorded onto a recording medium or the like. In the endoscope system 1 of the present embodiment, each time a frame image is conveyed, a frame image maximum in contrast evaluation value is determined. Thus, a quality still image can be displayed swiftly.

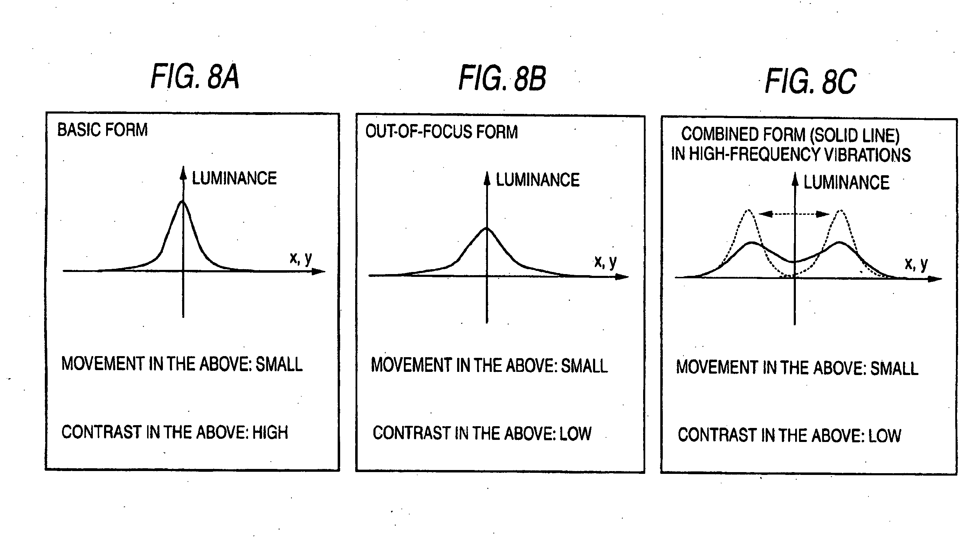

Fig. 8 is a figure for explaining the quality of the still image frozen by the endoscope system 1 of the present embodiment.

In Figs. 8A-8C, the horizontal axis represents a position of a target object in the frame image while the vertical axis represents a luminance in each position. Fig. 8A is a basic graph that a target object is photographed in a stationary state. In the state the target object is stationary, a clear luminance peak exists in the position of the target object.

In case the target object deviates in position due to beat, etc., the graph in Fig. 8A shifts, as it is, in the direction of the horizontal axis. In the related-art endoscope system that subject movement is detected to select a frame image least in movement, the deviation at the luminance peak of the graph shown in Fig. 8A is detected to select a frame image smallest in the deviation amount as a still image.

Fig. 8B shows a graph in a state out-of-focus is occurring in the frame image due to the deviation in depth distance between the target object and the optical probe 10 (CCD 133). Because the target object is not horizontally deviated in the occurrence of out-of-focus, a luminance peak is caused in the same position as the Fig. 8A. For this reason, in the conventional method to detect a subject movement, there is a possibility of selecting a frame image placed out of focus as an optimal still image. In Fig. 8B, the luminance level is decreased in the peak so that the frame image entirety has a decreased contrast evaluation value. Accordingly, the endoscope system 1 in the present embodiment is allowed to positively avoid the disadvantage of selecting a frame image placed out of focus.

Fig. 8C shows a graph in high-frequency vibrations wherein the target object moves in a shorter time than the frame rate. In high-frequency vibrations, because the target object actually is moving in the direction of the horizontal axis but its period of movement is shorter than the frame rate, the frame image has a luminance peaks less moves. For this reason, in the conventional method of detecting a subject movement, it is impossible to detect an image deviation caused by high-frequency vibrations. As shown in Fig. 8C, because the contrast of the frame image decreases due to combining of high-frequency waves during high-frequency vibrations, the endoscope system 1 in the present embodiment is allowed to accurately detect an image blur owing to high-frequency vibrations.

As discussed so far, the endoscope system 1 in the present embodiment can select a quality frame image free of out-of-focus, etc. as a still image.

Here, although the foregoing explained the example that calculates a contrast evaluation value each time taking a frame image, the endoscope apparatus in the invention may previously store the frame image photographed so that calculating a contrast evaluation value and determining a maximum-valued frame image can be executed upon receiving an instruction from the user.

Meanwhile, although the foregoing explained the example that the image processing device for freezing a still image out of the frame images constituting a moving image is applied to an endoscope system, the image processing device may be applied to the ordinary digital video camera or the like.

Meanwhile, although the foregoing explained the example that displays a subject image highest in contrast out of the subject images having been taken in the past within a time with reference to a time of issuing a time trigger, the display section may display a subject image taken in the future within a time with reference to a time of issuing a time trigger or may display a subject image taken in the past and future within a time with reference to a time of issuing a time trigger.

**Claims**

1. An imaging apparatus comprising:

an imaging section that is adapted to repeatedly take an image of a subject to obtain a plurality of subject images;
a contrast calculating section (430) that is adapted to calculate a contrast of the subject image with respect to each of the plurality of subject images each time the subject image is obtained at the imaging section;
a storage section (510) that is adapted to store a certain number of subject images in a newer order among the subject images obtained at the imaging section;
a time trigger generating section (122) that is adapted to receive an operation by a user during repeatedly taking the subject image at the imaging section and to issue a time trigger representing a time at which the operation is received;
a subject image selecting section (450) that is adapted to select, each time the contrast is calculated by the contrast calculating section, a subject image highest in the contrast among the subject images stored in the storage section as a candidate for a subject image to be displayed on the display section, and that is adapted to determine, when the time trigger is issued from the time trigger section, the subject image selected as the candidate for a subject image to be displayed on the display section, and
a display section (40) adapted to display the subject image determined by the subject image selecting section.

2. The imaging apparatus according to claim 1, wherein the display section (40) is adapted to display each time a subject image is obtained by the imaging section the obtained subject image, and the display section is adapted to display when the time trigger is issued from the time trigger generating section the subject image highest in the contrast among the part of the subject images.

3. The imaging apparatus according to claim 1, wherein the contrast calculating section (430) is adapted to obtain contrasts at a plurality of points in a subject image and calculates a contrast of the subject image based on the obtained contrasts.

4. The imaging apparatus according to claim 1, wherein the contrast calculating section (430) is adapted to obtain contrasts at a plurality of points in a subject image and calculates a contrast of the subject image based on contrasts equal to or greater than a lower limit out of the obtained contrasts.

5. The imaging apparatus according to claim 1, wherein the contrast calculating section (430) is adapted to obtain contrasts at a plurality of points in a subject image and calculates a contrast of the subject image based on the obtained contrasts after correcting those exceeding an upper limit to a value within the upper limit.

6. The imaging apparatus according to claim 1, wherein the imaging section is adapted to obtain an image of a subject having a light arrival area where subject light arrives from the subject and a light non-arrival area where the subject light does not arrive surrounding the light arrival area, and
the contrast calculating section (430) is adapted to calculate a contrast within the light arrival area as a contrast of the subject image.

7. An endoscope system comprising:

a light source that emits light;
a light conducting path that guides the light emitted from the light source and illuminates light to a subject; and
an imaging apparatus according to claim 1.


**Patentansprüche**

1. Bildgebungsvorrichtung, umfassend:

einen Bildgebungsteil, ausgebildet zum wiederholten Aufnehmen eines Bilds eines Subjekts, um eine Mehrzahl von Subjektbildern zu erhalten;
einen Kontrastberechnungsteil (430), ausgebildet zum Berechnen eines Kontrastes des Subjektbilds bezüglich jedes der mehreren Subjektbilder jedes Mal dann, wenn das Subjektbild an dem Bildgebungsteil erhalten wird;
einen Speicherteil (510), ausgebildet zum Speichern einer gewissen Anzahl von Subjektbildern in einer neueren Reihenfolge unter den an dem Bildgebungsteil erhaltenen Subjektbildern;

einen Auslösezeitpunkt-Erzeugungsteil (122), ausgebildet zum Empfangen einer Benutzerbetätigung während wiederholten Aufnehmens des Subjektbilds an dem Bildgebungsteil, und zum Ausgeben eines Auslösezeitpunktes, der eine Zeit repräsentiert, zu der die Benutzerbetätigung empfangen wird;

einen Subjektbild-Auswählteil (450), ausgebildet, um jedes Mal dann, wenn der Kontrast von dem Kontrastberechnungsteil berechnet wird, ein Subjektbild mit dem höchsten Kontrast unter den in dem Speicherteil gespeicherten Subjektbildern als Kandidaten für ein Subjektbild auszuwählen, welches von dem Anzeigeteil anzuzeigen ist, und weiterhin ausgebildet, um dann, wenn der Auslösezeitpunkt von dem Auslösezeitpunkt-Erzeugungsteil ausgegeben wird, das Subjektbild zu bestimmen, das als Kandidat für ein auf dem Anzeigeteil anzuzeigendes Subjektbild ausgewählt wurde, und

einen Anzeigeteil (40), der dazu ausgebildet ist, das Subjektbild anzuzeigen, welches von dem Subjektbild-Auswählteil bestimmt wurde.

2. Bildgebungsvorrichtung nach Anspruch 1, bei der der Anzeigeteil (40) dazu ausgebildet ist, jedes Mal dann, wenn von dem Bildgebungsteil ein Subjektbild erhalten wird, das erhaltene Subjektbild anzuzeigen, und der Anzeigeteil dazu ausgebildet ist, dann, wenn von dem Auslösezeitpunkt-Erzeugungsteil der Auslösezeitpunkt ausgegeben wird, dasjenige Subjektbild anzuzeigen, welches den höchsten Kontrast unter dem Teil der Subjektbilder aufweist.

3. Bildgebungsvorrichtung nach Anspruch 1, bei der der Kontrastberechnungsteil (430) dazu ausgebildet ist, Kontraste an mehreren Punkten innerhalb eines Subjektbilds zu ermitteln, und einen Kontrast des Subjektbilds basierend auf den ermittelten Kontrasten zu berechnen.

4. Bildgebungsvorrichtung nach Anspruch 1, bei der der Kontrastberechnungsteil (430) dazu ausgebildet ist, Kontraste an einer Mehrzahl von Punkten innerhalb eines Subjektbilds zu gewinnen, und einen Kontrast des Subjektbilds basierend auf Kontrasten zu berechnen, die gleich oder größer sind als eine Untergrenze der ermittelten Kontraste.

5. Bildgebungsvorrichtung nach Anspruch 1, bei der der Kontrastberechnungsteil (430) dazu ausgebildet ist, Kontraste an mehreren Punkten innerhalb eines Subjektbilds zu ermitteln, und einen Kontrast des Subjektbilds basierend auf den ermittelten Kontrasten zu berechnen, nach dem solche, die eine Obergrenze überschreiten, auf einen Wert innerhalb der Obergrenze korrigiert wurden.

6. Bildgebungsvorrichtung nach Anspruch 1, bei der der Abbildungsteil dazu ausgebildet ist, ein Bild eines Subjekts in einem Lichtaufnahmebereich zu erhalten, wo Subjektlicht von dem Subjekt aufgenommen wird, und einem kein Licht aufnehmenden Bereich zu erhalten, der den Lichtaufnahmebereich umgibt, und in welchem das Subjektlicht nicht ankommt, und

der Kontrastberechnungsteil (430) dazu ausgebildet ist, einen Kontrast innerhalb des Lichtaufnahmebereichs als Kontrast des Subjektbilds zu berechnen.

7. Endoskopsystem, umfassend:

eine Licht emittierende Lichtquelle;

einen Lichtleitpfad, der das von der Lichtquelle emittierte Licht leitet und ein Subjekt mit Licht beleuchtet; und

eine Bildgebungsvorrichtung nach Anspruch 1.

**Revendications**

1. Appareil de formation d'image comprenant :

une section de formation d'image qui est conçue pour prendre de manière répétée une image d'un sujet pour obtenir une pluralité d'images de sujet ;

une section de calcul de contraste (430) qui est conçue pour calculer un contraste de l'image de sujet en relation avec chacune de la pluralité d'images de sujet à chaque fois que l'image de sujet est obtenue au niveau de la section de formation d'image ;

une section de mémorisation (510) qui est conçue pour mémoriser un certain nombre d'images de sujet dans un ordre des plus récentes parmi les images de sujet obtenues au niveau de la section de formation d'image ;

une section de génération de déclenchement de temps (122) qui est conçue pour recevoir une opération par un utilisateur pendant la prise de manière répétée de l'image de sujet au niveau de la section de formation d'image et pour émettre un déclenchement de temps représentant un instant auquel l'opération est reçue ;
une section de sélection d'image de sujet (450) qui est conçue pour sélectionner, à chaque fois que le contraste est calculé par la section de calcul de contraste, une image de sujet ayant le contraste le plus élevé parmi les images de sujet mémorisées dans la section de mémorisation en tant que candidat pour une image de sujet à afficher sur la section d'affichage, et qui est conçue pour déterminer, lorsque le déclenchement de temps est émis par la section de déclenchement de temps, l'image de sujet sélectionnée en tant que candidat pour une image de sujet à afficher sur la section d'affichage, et
une section d'affichage (40) conçue pour afficher l'image de sujet déterminée par la section de sélection d'image de sujet.

2. Appareil de formation d'image selon la revendication 1, dans lequel la section d'affichage (40) est conçue pour afficher, à chaque fois qu'une image de sujet est obtenue par la section de formation d'image, l'image de sujet obtenue, et la section d'affichage est conçue pour afficher, lorsque le déclenchement de temps est émis par la section de génération de déclenchement de temps, l'image de sujet ayant le contraste le plus élevé parmi la partie des images de sujet.

3. Appareil de formation d'image selon la revendication 1, dans lequel la section de calcul de contraste (430) est conçue pour obtenir des contrastes en une pluralité de points dans une image de sujet et calcule un contraste de l'image de sujet sur la base des contrastes obtenus.

4. Appareil de formation d'image selon la revendication 1, dans lequel la section de calcul de contraste (430) est conçue pour obtenir des contrastes en une pluralité de points dans une image de sujet et calcule un contraste de l'image de sujet sur la base des contrastes supérieurs ou égaux à une limite inférieure parmi les contrastes obtenus.

5. Appareil de formation d'image selon la revendication 1, dans lequel la section de calcul de contraste (430) est conçue pour obtenir des contrastes en une pluralité de points dans une image de sujet et calcule un contraste de l'image de sujet sur la base des contrastes obtenus après correction de ceux dépassant une limite supérieure à une valeur inférieure à la limite supérieure.

6. Appareil de formation d'image selon la revendication 1, dans lequel la section de formation d'image est conçue pour obtenir une image d'un sujet ayant une zone d'arrivée de lumière dans laquelle une lumière de sujet arrive du sujet et une zone de non arrivée de lumière dans laquelle la lumière de sujet n'arrive pas entourant la zone d'arrivée de lumière, et
la section de calcul de contraste (430) est conçue pour calculer un contraste dans la zone d'arrivée de lumière en tant que contraste de l'image de sujet.

7. Système endoscopique comprenant :

une source de lumière qui émet une lumière ;
un trajet de conduction de lumière qui guide la lumière émise par la source de lumière et rayonne la lumière vers un sujet ; et
un appareil de formation d'image selon la revendication 1.

FIG. 1

FIG. 2

## FIG. 3

EP 2 053 862 B1

# FIG. 4

```
                    ( START )
                        │
                        ▼
              ┌──────────────────────┐
              │ ATTACH OPTICAL PROBE │──── (S10)
              └──────────────────────┘
    ┌──────────────────▼
    │                   │                    (S11)
    │          ◇─────────────────◇  NO
    │          ◇  IMAGE INPUTTED? ◇────────────┐
    │          ◇─────────────────◇             │
    │                  │ YES      (S12)         │
    │      NO ◇─────────────────◇              │
    │    ┌────◇  EVALUATION FRAME? ◇           │
    │    │    ◇─────────────────◇              │
    │    │            │ YES ◀──────────────┐   │
    │    │            │              (S13) │   │
    │    │   NO ◇──────────────────────────◇  │
    │    │ ┌───◇ SUBJECT-OF-ADDITION PIXEL? ◇ │
    │    │ │  ◇──────────────────────────◇    │
    │    │ │          │ YES                     │
    │    │ │  ┌──────────────────┐             │
    │    │ │  │ CALCULATE CONTRAST│──(S14)     │
    │    │ │  └──────────────────┘             │
    │    │ │          ▼                         │
    │    │ │  ┌──────────────────┐             │
    │    │ │  │  CORRECT CONTRAST │──(S15)     │
    │    │ │  └──────────────────┘             │
    │    │ │          ▼                         │
    │    │ │  ┌──────────────────┐             │
    │    │ │  │   ADD CONTRAST    │──(S16)     │
    │    │ │  └──────────────────┘             │
    │    │ └─────────►│                         │
    │    │            ▼              (S17)      │
    │    │   ◇──────────────────────◇  NO     │
    │    │   ◇ ALL PIXELS COMPLETED? ◇─────────┘
    │    │   ◇──────────────────────◇
    │    │            │ YES
    │    │  ┌────────────────────────┐
    │    │  │ UPDATE EVALUATION MEMORY│──(S18)
    │    │  └────────────────────────┘
    │    │            ▼
    │    │  ┌────────────────────────┐
    │    │  │ DETERMINE MAXIMUM VALUE │──(S19)
    │    │  └────────────────────────┘
    │    │            ▼               (S20)
    │    │   NO ◇─────────────────◇
    │    └──────◇  TRIGGER INPUT?  ◇
    │           ◇─────────────────◇
    │                   │ YES
    │          ┌─────────────────────┐
    │          │ DISPLAY STILL IMAGE │──(S21)
    │          └─────────────────────┘
    │                   ▼
    │                 ( END )
```

FIG. 5

FIG. 6A

FIG. 6B

# FIG. 7

EVALUATION
VALUE
UPDATE

510

0
1 } 511
2
3
4
5

⋮

10
11
12
13
14
15

MAXIMUM FRAME
CALCULATION

520

FRAME No.
RECORD

## FIG. 8A

BASIC FORM

LUMINANCE

x, y

MOVEMENT IN THE ABOVE: SMALL

CONTRAST IN THE ABOVE: HIGH

## FIG. 8B

OUT-OF-FOCUS FORM

LUMINANCE

x, y

MOVEMENT IN THE ABOVE: SMALL

CONTRAST IN THE ABOVE: LOW

## FIG. 8C

COMBINED FORM (SOLID LINE)
IN HIGH-FREQUENCY VIBRATIONS

LUMINANCE

x, y

MOVEMENT IN THE ABOVE: SMALL

CONTRAST IN THE ABOVE: LOW

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007275582 A
- JP 2902662 B
- JP 8034577 B
- US 20070156021 A1